# EUROPÄISCHE PATENTANMELDUNG

(11) **EP 1 759 632 A1**
(43) Veröffentlichungstag der Anmeldung: **07.03.2007**
(21) Anmeldenummer: 06014437.5
(22) Anmeldetag: 12.07.2006
(51) Int. Cl.: A61B 5/0215, A61B 17/00, A61B 5/00, A61B 5/07

(54) **Implantierbare Vorrichtung zur Messung biometrischer Parameter des Blutes**

(30) Priorität: 31.08.2005 DE 102005041208
(71) Anmelder: Osypka, Peter, Dr.-Ing., 79618 Rheinfelden-Herten (DE)
(72) Erfinder: Osypka, Peter, Dr.-Ing., 79618 Rheinfelden-Herten (DE)
(74) Vertreter: Maucher, Wolfgang

(57) **Zusammenfassung**

Eine unter der Außenseite eines Herzens (2) implantierbare Vorrichtung (1) zur Messung biometrischer Parameter des Blutes wie beispielsweise des Blutdrucks weist wenigstens einen telemetrisch oder mittels Transpondertechnik auswertbaren Sensor (3) auf, der an oder in einem Gehäuse (4) angeordnet ist, wobei zu der Vorrichtung (1) auch eine Halterung zur Fixierung des Sensors (3) oder seines Gehäuses (4) beispielsweise im Inneren einer Herzwand gehört. Als Halterung dient ein in oder durch die Außenwand (5) des Herzens (2) und/oder die Zwischenwand (6) durchstechbarer stiftartiger Anker (7), der an einem Zugelement (8) oder Faden befestigt ist, welcher Faden in Gebrauchsstellung mit dem Sensor (3) oder einem Gehäuse (4) in geeigneter Weise unmittelbar oder über eine in Längsrichtung verlaufende Öffnung (13) verbunden ist. Der Anker ist so ausgebildet, dass er einerseits in einer Punktionskanüle (10) aufgenommen und andererseits mit Hilfe eines Werkzeugs (9) oder Stiletts relativ zu dieser Punktionskanüle (10) und/oder durch die Herzwand (5) schiebbar oder stechbar ist. In Gebrauchsstellung ist an dem am entgegengesetzten Ende des Gehäuses (4) angreifenden Ende des Zugelements (15) ein Gegenanker (12), beispielsweise ein Knoten vorgesehen (Fig. 4).

## Beschreibung

Die Erfindung betrifft eine unter der Ober- oder Außenfläche eines Herzens implantierbare Vorrichtung zur Messung biometrischer Parameter des Blutes wie Blutdruck, Blutzusammensetzung, pH-Wert des Blutes, Blutzucker, Temperatur oder dergleichen mit wenigstens einem telemetrisch oder mittels Transpondertechnik auswertbaren Sensor und mit einer Halterung zur Fixierung des Sensors oder eines ihn enthaltenden Gehäuses am oder im Herzen.

Eine derartige Vorrichtung ist aus EP 1 050 265 A2 bekannt. Als Halterung für einen Sensor ist dabei ein expandierbarer Stent vorgesehen, der in einer chirurgisch anbringbaren Öffnung an der Herzinnenwand anbringbar ist. Dazu ist also erforderlich, die Herzinnenwand chirurgisch mit einer Öffnung zu versehen, was neben der aufwendigen Operation auch für die Herzfunktion nachteilig ist.

Es besteht deshalb die Aufgabe, eine Vorrichtung der eingangs genannten Art zu schaffen, mit welcher der Sensor trotz der Bewegung des Herzens mit großer Sicherheit an diesem befestigt werden kann, ohne die Herzleistung nachteilig zu beeinträchtigen.

Zur Lösung dieser Aufgabe ist die eingangs definierte implantierbare Vorrichtung dadurch gekennzeichnet, dass als Halterung für den Sensor oder sein Gehäuse ein von der Außenseite des Herzens aus in oder durch die Außenwand des Herzens und / oder die Zwischenwand durchstechbarer stiftartiger Anker vorgesehen ist, der an einem Zugelement oder Faden befestigt und zumindest in Gebrauchsstellung mit dem Sensor verbunden ist, wobei der Faden oder das Zugelement zwischen den beiden Enden des stiftartigen Ankers an diesem angreift, und dass der Anker mittels eines Werkzeugs oder Stiletts von außen in oder durch die Herzwand schiebbar oder stechbar ist.

Auf diese Weise ergibt sich eine von der Außenseite des Herzens aus implantierbare Vorrichtung mit einem Sensor, die innerhalb der Herzaußenwand oder der Herzzwischenwand oder auch im Inneren einer Herzkammer befestigt werden kann, was mit Hilfe des Ankers und des Zugelements oder Fadens lagegenau durchgeführt werden kann, dennoch aber nur eine ganz geringe Verletzung bewirkt. Gleichzeitig kann sich diese Befestigung gut an die Bewegungen des Herzens anpassen. Ein weiterer Vorteil besteht darin, dass der Sensor auch relativ einfach wieder entfernt werden kann, indem lediglich der Anker von seinem Zugelement abgetrennt und dann der Sensor an der diesem Anker abgewandten Seite wieder nach außen herausgezogen wird. Eine aufwändige Operation und die Installation eines Stents werden vermieden.

Besonders zweckmäßig ist es dabei, wenn der Anker relativ zu dem Zugelement um dessen Befestigungsstelle an ihm schwenkbar ist. Somit kann der Anker nach dem Platzieren selbsttätig eine Position quer zu der Stelle einnehmen, durch welche er von außen hindurchgeschoben oder hindurchgestochen wurde, so dass danach das Zugelement bereits entsprechende Zug- und Haltekräfte übertragen kann.

Als Angriffsstelle für das Werkzeug oder Stilett an dem Anker kann eine Verformung an dem der durchstechbaren Spitze oder dem durchstechbaren Ende entgegengesetzten Ende angeordnete Verformung vorgesehen sein, die mit dem Werkzeug oder Stilett lösbar kuppelbar ist. Somit kann der Anker durch das Werkzeug oder Stilett formschlüssig umgriffen oder erfasst und in axialer Richtung in seine Lage verschoben werden.

Der Anker kann an seiner in Einführrichtung rückwärtigen, der Spitze oder dem stirnseitigen Ende abgewandten Seite eine Querschnittsverminderung für ein aufsteckbares Ende eines Werkzeuges und / oder eine an diesem Ende offene und am entgegengesetzten Ende geschlossene Aufnahmeöffnung für ein in diese Aufnahmeöffnung passendes Werkzeug oder Stilett haben, womit der Anker in seine Gebrauchsstellung verschiebbar ist. Auf einfache Weise lässt sich so der Anker für die Implantation formschlüssig mit einem Werkzeug oder Stilett verbinden.

Dabei kann der Vorrichtung als Hilfsgerät für die Implantation eine Punktionskanüle zugehören, deren Innenhöhlung zumindest an ihrem äußeren oder distalen Ende etwa dem Außenquerschnitt des Ankers entspricht und den Anker zumindest teilweise in sich aufnimmt, wobei der Faden oder das Zugelement in dieser Lage an der Außenseite der Punktionskanüle verläuft. Mit dieser Punktionskanüle kann also die Implantationsstelle für den Anker und damit auch für den Sensor vorbereitet werden, indem die Herzwand entsprechend punktiert und dann der Anker mit dem Zugelement als Halterung für den Sensor aus dieser Punktionskanüle durch die von ihr geschaffene Öffnung hindurch verschoben und in Gebrauchsstellung gebracht wird.

Zweckmäßig ist es, wenn das Gehäuse des Sensors eine etwa zylindrische Querschnittsform mit wenigstem einem, insbesondere zwei abgerundeten und / oder angespitzten Enden hat, wobei das Zugelement zumindest in Gebrauchsstellung an oder nach einem oder beiden Enden des Gehäuses angreift. Somit kann der Sensor an nahezu beliebigen Stellen der Herzwand innerhalb dieser oder auch durch die Herzwand hindurch im Inneren einer Herzkammer platziert und durch das an ihm angreifende Zugelement gehalten werden. Die zylindrische Querschnittsform erleichtert dabei das Einschieben dieses Sensors in die zuvor von dem Anker und der Punktionskanüle von außen her geschaffene Öffnung bis zu der Stelle, wo der Sensor platziert sein soll.

Bei einer zweckmäßigen Ausführungsform kann das Gehäuse des Sensors oder der Sensor selbst eine in seiner Längsrichtung verlaufende Öffnung haben, mittels welcher er über den Faden oder das Zugelement des Ankers verschiebbar ist, sodass der Sensor nach der Installation des Ankers über den Faden in seine Gebrauchslage verschiebbar ist. Es kann also zunächst der Anker implantiert und dann über dessen Zugelement der Sensor in seine gewünschte Lage verschoben und dort mit Hilfe des Zugelements fixiert werden. Dabei kann die Verschiebbarkeit des Sensors auf dem Zugelement schwergängig sein, sodass er schon durch Reibung an dem Zugelement gehalten wird, falls er im Inneren einer Herzkammer platziert wird. Bei einer Verschiebung in eine Lage innerhalb der Herzwand wird der Sensor von dem ihn umschließenden Gewebe genügend festgelegt, so dass auch eine leichtgängige Verschiebung auf dem Zugelement möglich ist.

Das in Gebrauchsstellung über den Sensor an der dem Anker abgewandten Seite überstehende Ende des Zugelements oder Fadens kann zur Gegenhalterung beispielsweise an der Herzaußenseite mit einem Gegenanker oder Knoten versehen sein. Der einfachste Gegenanker ist dabei der Knoten, den der Chirurg nach dem Einführen des Sensors an dem außen am Herzen überstehenden Ende des Fadens oder Zugelements anbringen kann. Ebenso kann er dort aber auch einen Gegenanker fixieren, wodurch das Zugelement mit dem von vorne herein an ihm befindlichen Anker einerseits und diesem Gegenanker andererseits festgelegt ist, so dass der dazwischen befindliche Sensor in der gewünschten Lage gehalten ist und gehalten bleibt, obwohl das Herz Bewegungen durchführt.

Eine weitere zweckmäßige Ausgestaltung der Erfindung kann vorsehen, dass der auf das Zugelement aufschiebbare Sensor einen zusätzlichen Faden trägt, mittels welchem er auch entgegen der Schieberichtung positionierbar oder wieder aus dem Herzen herausziehbar ist.

Eine abgewandelte Ausführungsform kann vorgesehen, dass an der einen Seite des Sensors, insbesondere an einem abgerundeten oder angespitzten Ende, das Zugelement mit dem Anker und an der - in Einführrichtung - entgegengesetzten Seite das Zugelement für den Gegenanker oder Knoten angreift. Somit wird schon beim Implantieren des Ankers und seines Zugelements der Sensor "mitgenommen", wobei der Anker so zu platzieren ist, dass der Sensor seine vorgesehene Position bevorzugt innerhalb einer Herzkammer erhält. Danach braucht nur noch das zweite Zugelement mit dem Gegenanker oder einem Knoten versehen oder befestigt zu werden.

Das Gehäuse des Sensors kann aus biokompatiblem Werkstoff bestehen und im Verhältnis zu seiner Länge einen geringen Querschnitt haben. Dadurch kann es gut durch eine zuvor punktierte Stelle der Herzwand hindurchverschoben werden, sei es beim Implantieren, sei es auch später wieder beim Entfernen.

In dem Gehäuse des bevorzugt als Drucksensor ausgebildeten Sensors können wenigstens zwei Druckaufnehmer angeordnet sein, die zueinander beabstandet oder einander gegenüberliegend in oder an dem Gehäuse angeordnet sind. Ein solcher Sensor ist vor allem zur Implantation in der Herzzwischenwand geeignet, wo er mit den beiden Druckaufnehmern den Blutdruck sowohl in der linken als auch in der rechten Herzkammer ermitteln und/oder überwachen kann.

Vor allem bei Kombination einzelner oder mehrerer der vorbeschriebenen Merkmale und Maßnahmen ergibt sich eine Vorrichtung zur Messung biometrischer Parameter des Blutes, die auf einfache Weise von außen unter der Ober- oder Außenfläche im Herzen implantiert und gehaltert werden kann, ohne dass ein großer chirurgischer Eingriff zur Anbringung einer entsprechenden Halteöffnung für die Halterung des Sensors in der Herzzwischenwand notwendig ist. Vielmehr kann die im wesentlichen aus einem Zugelement und einem an dessen freien Ende angeordneten Anker bestehende Halterung für den Sensor mittels einer Punktionskanüle und einem Stilett in oder an der Herzwand angebracht und damit oder dadurch der Sensor platziert und festgelegt werden. Dabei kann er entweder im Inneren der Herzwand oder Herzzwischenwand oder auch zwischen den eine Herzkammer begrenzenden Wänden angeordnet werden. Aufgrund dieser einfachen Implantierbarkeit, die sich auch auf das Herz selber praktisch nicht auswirkt und bei welcher auch der Sensor am Herzen nur einen minimalen Platzbedarf hat, können gegebenenfalls auch mehrere derartige Sensoren implantiert werden, um entsprechend viele biometrische Parameter des Blutes im und am Herzen erfassen zu können.

Nachstehend sind Ausführungsbeispiele der Erfindung anhand der Zeichnung näher beschrieben. Es zeigt in zum Teil erheblich schematisierter Darstellung:
- Fig. 1: eine implantierbare Vorrichtung zur Messung biometrischer Parameter des Blutes in Form eines Gehäuses eines Sensors und einer als Zugelement oder Faden ausgebildeten Halterung, wobei an dem dem Sensor abgewandten Ende des Zugelements ein Anker angeordnet ist, der dabei innerhalb einer Punktionskanüle für seine Implantation eingeschoben ist,
- Fig. 2: im vergrößertem Maßstab des Sensor und das mit einem Anker versehene Zugelement, wobei der Anker relativ zu dem Zugelement um dessen Befestigungsstelle an ihm in die Gebrauchslage quer verstellt ist, wie es auch in Gebrauchsstellung vorgesehen ist,
- Fig. 3: das auch in Fig. 1 im Inneren der Punktionskanüle angedeutete Werkzeug bei seinem Angriff an dem Anker, womit er nach dem Punktieren der vorgesehenen Stelle eines Herzens aus der Punktionskanüle in Gebrauchslage ausschiebbar ist,
- Fig. 4: die Spitze eines Herzens im Längsschnitt, wobei man die beiden Herzkammern und die Herzzwischenwand erkennt und die Punktionskanüle die Herzaußenseite über einen Teil der Länge durchstoßen hat,
- Fig. 5: eine der Fig. 4 entsprechende Darstellung nach dem Ausschieben des Ankers aus der Punktionskanüle, wodurch er an der Herzaußenseite zu liegen kommt, während das Zugelement durch den zuvor von der Punktionskanüle geschaffenen Kanal verläuft und ein gegenüber den Fig. 1 bis 3 abgewandelter Sensor in einem Gehäuse angeordnet ist, welches eine in Längsrichtung verlaufende Öffnung hat, mittels welcher der Sensor mit Hilfe eines Katheters über den Faden oder das Zugelement des Ankers verschiebbar ist, wobei der Sensor noch außerhalb des Herzens angeordnet ist,
- Fig. 6: eine den Fig. 4 und 5 entsprechende Darstellung nach dem Verschieben des Sensors bis in das Innere der Herzaußenwand, wobei das dem Anker entgegengesetzte Ende des Zugelements mit einem Gegenanker versehen und festgelegt ist,
- Fig. 7: im vergrößertem Maßstab das Gehäuse eines Sensors mit einem Sensor und einer in Längsrichtung verlaufenden Öffnung sowie einem an dem Gehäuse angreifenden Zugelement gemäß Fig. 5.
- Fig. 8: eine abgewandelte Ausführungsform eines implantierbaren Sensors mit einem Drucksensor, bei welchem an beiden Enden des länglichen Gehäuses jeweils ein Zugelement angreift und das in Einführrichtung vor dem Sensor befindliche Zugelement den Anker aufweist, wie es etwa Fig. 1 und 2 entspricht,
- Fig. 9: eine Ausführungsform, bei welcher das Gehäuse zwei einander gegenüberliegende Drucksensoren aufweist und eine durchgehende Öffnung sowie ein am in Vorschubrichtung hinteren Ende angreifendes Zugelement aufweist analog der Ausführungsform nach Fig. 7,
- Fig. 10: eine der Fig. 8 entsprechende Ausführungsform, bei welcher die Zugelemente unmittelbar an dem Gehäuse nach entgegengesetzten Richtungen angreifend angeordnet sind und das in Einführrichtung verlaufende Zugelement den Anker aufweist, wobei das Gehäuse zwei Sensoren enthält, sowie
- Fig. 11 bis 16: schematisierte Beispiele, wie und wo der Sensor implantiert und mit seiner Halterung in Form eines Zugelements und Ankers festgelegt sein kann.

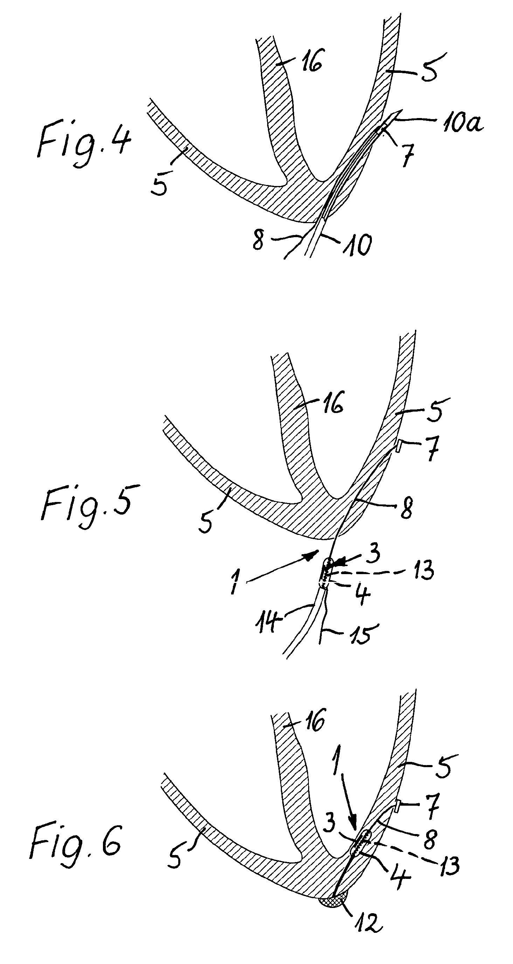

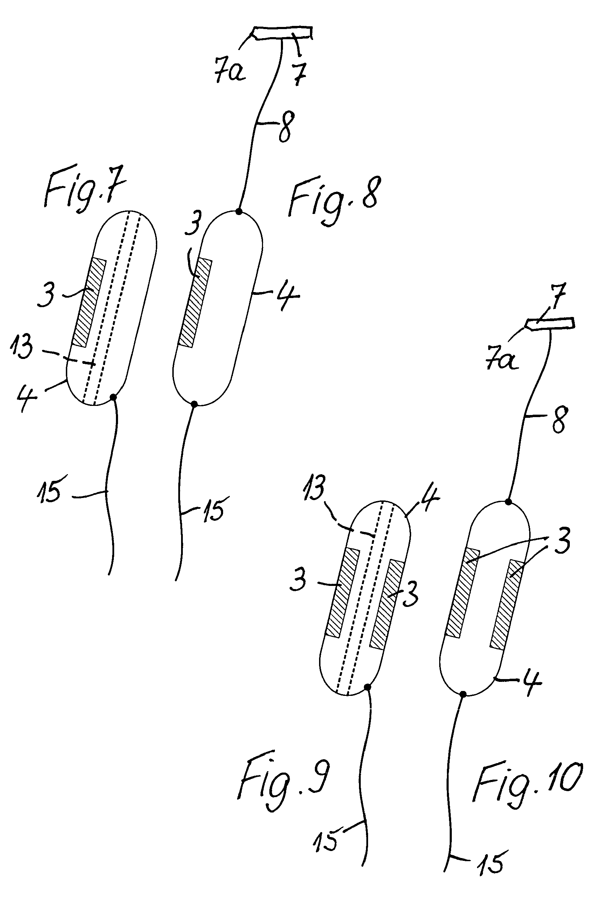

Eine im Ganzen mit 1 bezeichnete, gemäß den Fig. 4 bis 6 und 11 bis 16 von außen her unter der Ober- oder Außenfläche eines Herzens 2 implantierbare Vorrichtung dient zur Messung biometrischer Parameter des Blutes, beispielsweise des Blutdruckes, aber auch anderer Parameter und weist wenigstens einen (Fig. 7 und 8) oder zwei (Fig. 9 und 10) telemetrisch oder mittels Transpondertechnik auswertbare Sensoren 3 und eine noch näher zu erläuternde Halterung zur Fixierung des jeweiligen Sensors oder eines ihn enthaltenden Gehäuses 4 am oder im Herzen 2 auf.

Als Halterung für den Sensor 3 oder sein Gehäuse 4 ist in den Ausführungsbeispielen ein in oder durch die Außenwand 5 des Herzens 2 und/oder die Zwischenwand 6 dieses Herzens 2 durchstechbarer stiftartiger Anker 7 vorgesehen, der an einem Zugelement 8 oder Faden befestigt ist und mit diesem zusammen die Halterung bildet, die zumindest in Gebrauchsstellung mit dem Sensor 3 oder seinem Gehäuse 4 verbunden ist, wie es im Folgenden noch näher erläutert wird.

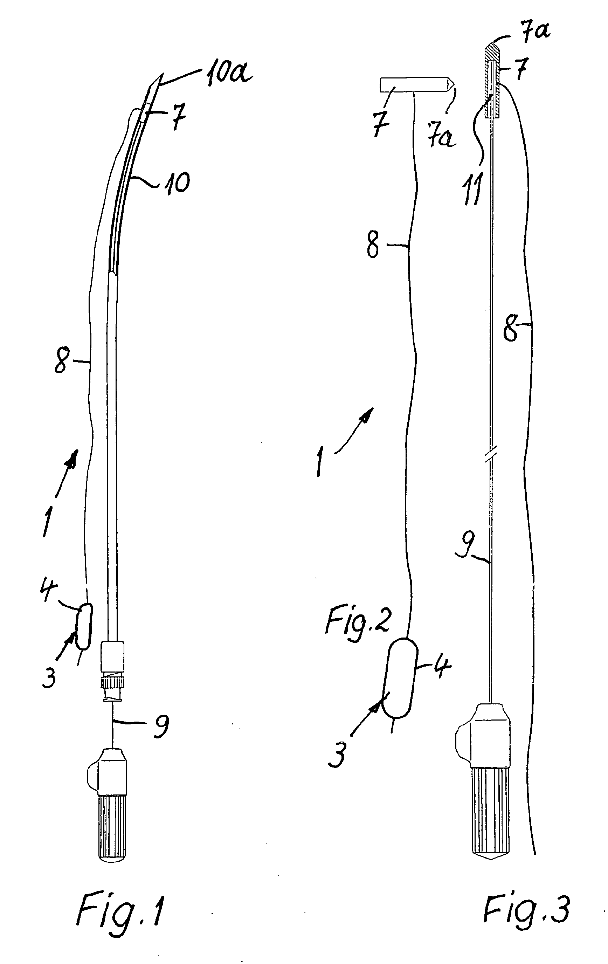

Gemäß Fig. 1 bis 3 greift dabei der Faden oder das Zugelement 8 zwischen den beiden Enden des stiftartigen Ankers 7 an diesem an, so dass er sich in Gebrauchsstellung analog Fig. 2 quer zu dem Zugelement 8 stellen und damit die Lage des Zugelements 8 fixieren kann, wie es beispielsweise in Fig. 5 sowie auch weiteren Zeichnungen von Ausführungsbeispielen deutlich erkennbar ist. Der Anker 7, welcher an dem in Einsteckrichtung vorderen Ende einer Spitze 7a hat, ist dabei gemäß Fig. 1 und 3 mittels eines Werkzeuges 9 oder Stiletts in oder durch die Herzwand 5 schiebbar oder stechbar. Dabei ist der Anker 7 aufgrund der Nachgiebigkeit des Zugelements 8 relativ zu diesem um die Befestigungsstelle schwenkbar, wie es der Vergleich der Fig. 2 und 3 verdeutlicht. Als Angriffsstelle für das Werkzeug oder Stilett 9 ist dabei an dem Anker 7 eine Verformung an deren der durchstechbaren Spitze 7a entgegengesetzten Ende vorgesehen, die mit dem Werkzeug 9 oder Stilett lösbar kuppelbar ist. Im Ausführungsbeispiel hat der Anker 7 an seiner in Einführrichtung rückwärtigen, der Spitze 7a abgewandten Seite eine an diesem offene und am entgegengesetzten Ende geschlossene Aufnahmeöffnung 11 für das in diese Aufnahmeöffnung passende Werkzeug oder Stilett 9, womit der Anker in seine Gebrauchsstellung verschiebbar beziehungsweise nach dem Einführen mit Hilfe einer noch zu erläuternden Punktionskanüle 10 beim Zurückziehen dieser Punktionskanüle 10 festgehalten werden kann, um diese Gebrauchsstellung dann endgültig nach dem Zurückziehen auch des Werkzeugs 9 einzunehmen.

Gemäß Fig. 1 gehört als Hilfsgerät für die Implantation zu der Vorrichtung 1 die schon erwähnte Punktionskanüle 10, deren Innenhöhlung zumindest an ihrem äußeren oder distalen Ende 10a etwa des Außenquerschnitt des Ankers 7 entspricht oder diesen übertrifft und den Anker 7 zumindest teilweise oder gemäß Fig. 1 und 4 vollständig in sich aufnimmt, wobei der Faden oder Zugelement 8 in dieser Lage an der Außenseite der Punktionskanüle 10 verläuft.

Mit der Vorrichtung 1 und dabei zunächst ihrer Punktionskanüle 10 kann also die Halterung des Sensors 3 oder seines Gehäuses 4, nämlich der Anker 7 und das Zugelement 8 durch die Herzwand 5 hindurchtransportiert werden. Hat der Anker 7 wieder die Außenseite des Herzens 2 und der Herzwand 5 erreicht, wird das Werkzeug 9 in dieser Lage festgehalten, aber die Punktionskanüle 10 zurückgezogen, so dass nunmehr der Anker 7 an der Außenseite des Herzens 2 platziert ist, während das Zugelement 8 durch die Herzwand 5 zurückverläuft. Ist dabei gemäß Fig. 1 und 2 das Gehäuse 4 des Sensors in zutreffender Entfernung zu dem Anker 7 angeordnet, befindet sich dieses dann zusammen mit dem Zugelement 8 ebenfalls innerhalb der Herzwand 5. Es braucht dann nur noch beispielsweise gemäß Fig. 6 ein Gegenanker 12 an dem dem Anker 7 abgewandten Ende des Zugelements 8 angebracht zu werden, wobei dieser Gegenanker 12 auch ein Knoten des Zugelements 8 sein kann, um das Gehäuse 4 des Sensors 3 beispielsweise innerhalb der Herzwand 5 zu fixieren.

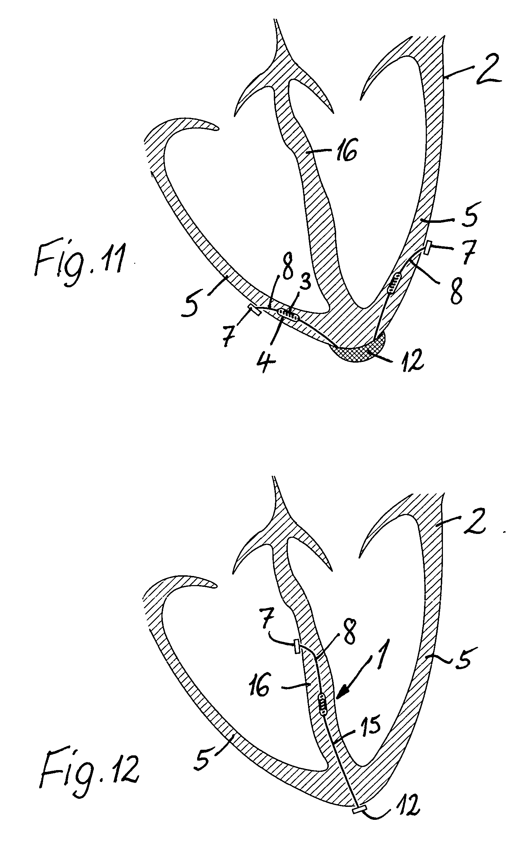

Gemäß den Fig. 7 bis 10 hat das Gehäuse 4, welches den Sensor 3 enthält, eine etwa zylindrische Querschnittsform mit zwei abgerundeten Enden, die das Einführen des Gehäuses 4 in eine durch die Punktionskanüle 10 geschaffene Öffnung am Herzen erleichtert. Gegebenenfalls könnten diese Enden auch angespitzt sein. Gemäß den Fig. 8 und 10 greift das Zugelement 8 an den beiden Enden des Gehäuses 4, so dass es beispielsweise gemäß Fig. 11 platziert werden kann.

Eine abgewandelte Ausführungsform zeigt Fig. 7 und 9 und sieht vor, dass das Gehäuse 4 des oder der Sensoren 3 eine in seiner Längsrichtung verlaufende Öffnung 13 hat, die durchgängig ist und mit welcher das Gehäuse 4 über den Faden oder Zugelement 8 des Ankers 7 verschiebbar ist, so dass also der Sensor 3 nach der Installation des Ankers 7 gemäß Fig. 5 über diesen Faden 8 in seine Gebrauchslage mit Hilfe eines Katheters 14 verschiebbar ist.

Es ist also entweder möglich, das Gehäuse 4 mit dem Sensor 3 unmittelbar durch das Installieren des Ankers 7 in seine Gebrauchslage zu bringen oder aber zunächst den Anker 7 mit seinem Zugelement 8 oder Faden an der Herzwand 5 zu befestigen und danach einen Sensor 3 mit Hilfe eines Gehäuses 4, welches eine Längsöffnung 13 hat, über den zur Halterung dienenden Faden 8 zu verschieben, der also gleichzeitig die Funktion eines Führungsfadens oder Führungsdrahtes hat, falls das Zugelement 8 aus einem Draht besteht. Auch in diesem Falle kann dann nach dem Verschieben des Gehäuses 4 über dieses Zugelement 8 in seine Gebrauchsstellung an der Außenseite des Herzens eine Gegenhalterung 12 durch Verknoten vorgesehen werden, weil der Sensor durch Reibung, sei es auf dem Zugelement 8, sei es im Gewebe des Herzens 2, ausreichend festgelegt ist.

In allen Ausführungsbeispielen hat das Gehäuse 4 auch einen entgegen der Einschubrichtung verlaufenden Faden oder ein Zugelement 15 und man erkennt in den Fig. 7 und 9, dass auch der auf das Zugelement 8 aufschiebbare und darauf verschiebbare Sensor 3 beziehungsweise sein Gehäuse einen solchen zusätzlichen Faden 15 trägt, mit welchem das Gehäuse 4 auch entgegen der Schieberichtung positionierbar oder wieder aus dem Herzen 2 herausziehbar ist. Ist also beispielsweise das Gehäuse 4 beim Implantieren mit Hilfe des Katheters 14 zu weit verschoben worden, kann durch eine Zugkraft an diesem zusätzlichen Zugelement oder Faden 15 das Gehäuse 4 wieder etwa zurückbewegt werden.

Im Ausführungsbeispiel nach Fig. 8 und 10 greift hingegen an der einen Seite des Sensors oder seines Gehäuses 4 das Zugelement 8 mit dem Anker 7 und an der entgegengesetzten Seite das Zugelement 15 für den Gegenanker oder Knoten an.

Dabei hat das Gehäuse 4 des Sensors 3 im Verhältnis zu seiner Länge einen relativ geringen Querschnitt, um gut über einen punktierten Kanal und/oder ein Zugelement 8 in dem Herzgewebe verschoben werden zu können. Gleichzeitig besteht das Gehäuse 4 aus biokompatiblen Werkstoff.

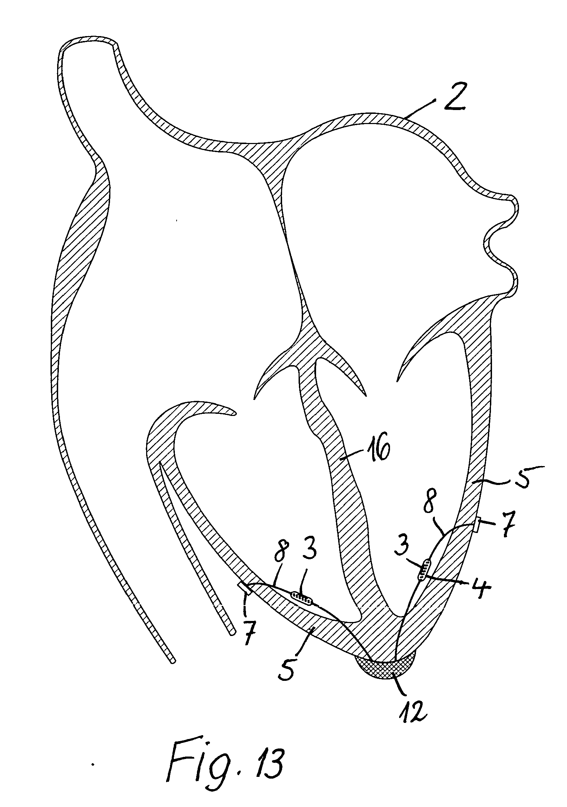

Während in den Ausführungsbeispielen gemäß Fig. 7 und 8 in dem Gehäuse 4 jeweils ein Sensor 3 angeordnet ist, zeigen die Ausführungsformen gemäß Fig. 9 und 10, dass in dem Gehäuse 4 auch wenigstens zwei Druckaufnehmer oder Sensoren 3 angeordnet sein können, die dabei zueinander beabstandet oder einander gegenüberliegend angeordnet sind. Eine solche Ausführungsform eignet sich besonders gut zur Implantierung in der Herzzwischenwand 16, wie es in Fig. 12 dargestellt ist, so dass dann der der rechten Herzkammer zugewandte Sensor den Druck in der rechten Herzkammer und der andere Sensor 3 den Druck in der linken Herzkammer messen oder überwachen kann.

Fig. 11 zeigt hingegen eine Anordnung, bei welcher in den Herzaußenwänden 5 der beiden Herzkammern jeweils ein Gehäuse 4 implantiert und mit Hilfe eines Zugelements 8 und eines Ankers 7 sowie eines gemeinsamen Gegenankers 12 festgelegt sind.

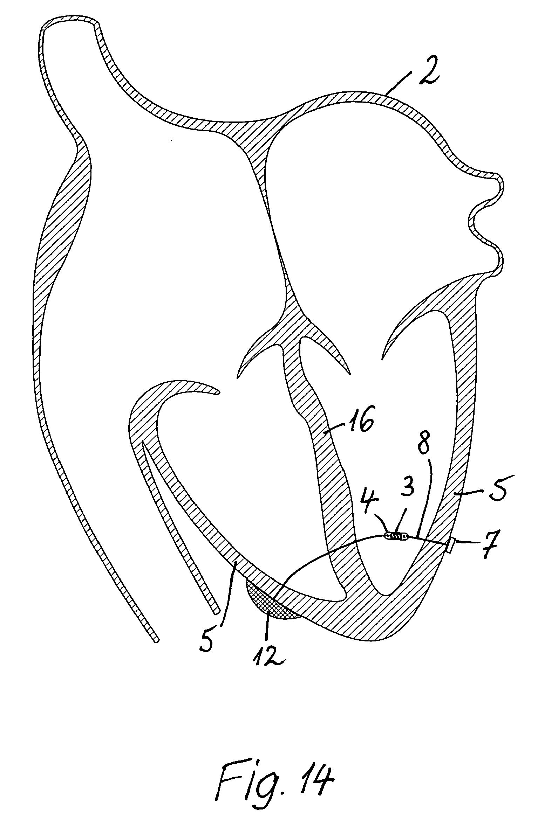

Gemäß Fig. 13 können bei einer analogen Verankerung, wie sie in Fig. 11 zu sehen ist, die beiden in den beiden Herzkammern befindlichen Sensoren 3 aber auch in diesen Herzkammern 3 gehalten sein, weil in diesem Falle die Zugelemente 8 die Herzkammern gewissermaßen durchqueren.

Eine vergleichbare Anordnung mit nur einem Sensor 3 in der linken Herzkammer zeigt Fig. 14, bei welcher die Zugelemente 8 und 15 beide Herzkammern durchqueren und jeweils außenseitig mit einem Anker 7 und einem Gegenanker 12 festgelegt sind, aber nur im Bereich der linken Herzkammer dieser Drucksensor angeordnet ist.

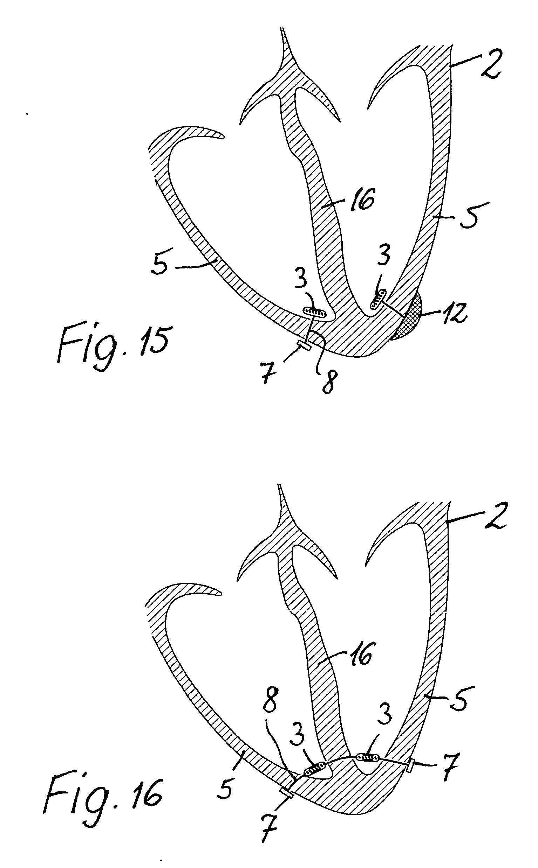

Fig. 15 zeigt in jeder Herzkammer einen Sensor oder ein Gehäuse 4, wobei Zugelemente die Herzaußenwand 5 durchqueren und dort mit einem Anker 7 oder einem Gegenanker 12 fixiert sind. Ein zunächst zwischen beiden Gehäusen 4 verlaufendes Zugelement kann aus einem selbstauflösenden Werkstoff bestanden haben und deshalb gemäß Fig. 15 verschwunden sein.

Fig. 16 zeigt ebenfalls eine Anordnung mit einem Zugelement, welches durch beide Herzkammern verläuft und jeweils außenseitig mit Ankern 7 fixiert ist, wobei auf dem Zugelement 8 in jeder Herzkammer ein Gehäuse 4 mit wenigstens einem Sensor angeordnet ist.

Weitere Anordnungen und Platzierungen auch von noch mehr derartigen Gehäusen 4 mit einem oder zwei oder gegebenenfalls noch mehr Sensoren 3 sind aufgrund der nur geringfügig ein Herz 2 beeinträchtigenden Halterung und der sehr einfachen Implantationsart möglich.

Die von außen aus unter der Außenseite eines Herzens 2 implantierbare Vorrichtung 1 zur Messung biometrischer Parameter des Blutes wie beispielsweise des Blutdrucks weist wenigstens einen telemetrisch oder mittels Transpondertechnik auswertbaren Sensor 3 auf, der an oder in einem Gehäuse 4 angeordnet ist, wobei zu der Vorrichtung 1 auch eine Halterung zur Fixierung des Sensors 3 oder seines Gehäuses 4 beispielsweise im Inneren einer Herzwand gehört. Als Halterung dient ein von der Außenseite des Herzens aus in oder durch die Außenwand 5 des Herzens 2 und/oder die Zwischenwand 6 durchstechbarer stiftartiger Anker 7, der an einem Zugelement 8 oder Faden befestigt ist, welcher Faden in Gebrauchsstellung mit dem Sensor 3 oder einem Gehäuse 4 in geeigneter Weise unmittelbar oder über eine in Längsrichtung verlaufende Öffnung 13 verbunden ist. Der Anker ist so ausgebildet, dass er einerseits in einer Punktionskanüle 10 aufgenommen und andererseits mit Hilfe eines Werkzeugs 9 oder Stiletts relativ zu dieser Punktionskanüle 10 und/oder durch die Herzwand 5 schiebbar oder stechbar ist. In Gebrauchsstellung ist an dem am entgegengesetzten Ende des Gehäuses 4 angreifenden Ende des Zugelements 15 ein Gegenanker 12, beispielsweise in kompakter Form oder als Knoten vorgesehen.

Es sei noch erwähnt, dass der Gegenanker 12 in der in den Fig. 6 und 11 bis 15 dargestellten kompakten Ausführung auch einen Teil der Elektronik des Sensors 3 enthalten und dann beispielsweise gehäuseartig ausgebildet sein kann.

## Patentansprüche

1. Unter der Ober- oder Außenfläche eines Herzens (2) implantierbare Vorrichtung (1) zur Messung biometrischer Parameter des Blutes, wie Blutdruck, Blutzusammensetzung, pH-Wert des Blutes, Blutzucker, Temperatur oder dergleichen mit wenigstens einem telemetrisch oder mittels Transpondertechnik auswertbaren Sensor (3) und mit einer Halterung zur Fixierung des Sensors (3) oder eines ihn enthaltenden Gehäuses (4) am oder im Herzen (2), **dadurch gekennzeichnet, dass** als Halterung für den Sensor (3) oder sein Gehäuse (4) ein von der Außenseite des Herzens (2) aus in oder durch die Außenwand (5) des Herzens (2) und/oder die Zwischenwand (6) durchstechbarer stiftartiger Anker (7) vorgesehen ist, der an einem Zugelement (8) oder Faden befestigt und zumindest in Gebrauchsstellung mit dem Sensor (3) verbunden ist, wobei der Faden oder das Zugelement (8) zwischen den beiden Enden des stiftartigen Ankers (7) an diesem angreift, und dass der Anker (7) mittels eines Werkzeugs (9) oder Stiletts von außen in oder durch die Herzwand (5) schiebbar oder stechbar ist.

2. Vorrichtung nach Anspruch 1, **dadurch gekennzeichnet, dass** der Anker (7) relativ zu dem Zugelement (8) um dessen Befestigungsstelle an ihm schwenkbar ist.

3. Vorrichtung nach einem der Ansprüche 1 bis 2, **dadurch gekennzeichnet, dass** als Angriffsstelle für das Werkzeug (9) oder Stilett an dem Anker (7) eine Verformung an dem der durchstechbaren Spitze (7a) oder dem durchstechbaren Ende entgegengesetzten Ende vorgesehen ist, die mit dem Werkzeug (9) oder Stilett lösbar kuppelbar ist.

4. Vorrichtung nach einem der Ansprüche 1 bis 3, **dadurch gekennzeichnet, dass** der Anker (7) an seiner in Einführrichtung rückwärtigen, der Spitze (7a) oder dem einstechbaren Ende abgewandten Seite eine Querschnittsverminderung für ein aufsteckbares Ende eines Werkzeuges (9) und/oder eine an diesem Ende offene und am entgegengesetzten Ende geschlossene Aufnahmeöffnung (11) für ein in diese Aufnahmeöffnung passendes Werkzeug (9) oder Stilett hat, womit der Anker (7) in seine Gebrauchsstellung verschiebbar ist.

5. Vorrichtung nach einem der Ansprüche 1 bis 4, **dadurch gekennzeichnet, dass** ihr eine Punktionskanüle (10) zugehört, deren Innenhöhlung zumindest an ihrem äußeren oder distalen Ende (10a) etwa dem Außenquerschnitt des Ankers (7) entspricht und den Anker (7) zumindest teilweise in sich aufnimmt, wobei der Faden oder das Zugelement (8) in dieser Lage an der Außenseite der Punktionskanüle (10) verläuft.

6. Vorrichtung nach einem der Ansprüche 1 bis 5, **dadurch gekennzeichnet, dass** das Gehäuse (4) des Sensors (3) eine etwa zylindrische Querschnittsform mit wenigstens einem, insbesondere zwei abgerundeten und/oder angespitzten Enden hat, wobei das Zugelement (8) zumindest in Gebrauchsstellung an oder nahe einem oder beiden Enden des Gehäuses (4) angreift.

7. Vorrichtung nach einem der Ansprüche 1 bis 6, **dadurch gekennzeichnet, dass** das Gehäuse (4) des Sensors (3) oder der Sensor eine in seiner Längsrichtung verlaufende Öffnung (13) hat, mittels welcher er über den Faden oder das Zugelement (8) des Ankers (7) verschiebbar ist, so dass der Sensor (3) nach der Installation des Ankers (7) über den Faden (8) in seine Gebrauchslage verschiebbar ist.

8. Vorrichtung nach einem der Ansprüche 1 bis 7, **dadurch gekennzeichnet, dass** das in Gebrauchsstellung über den Sensor an der dem Anker abgewandten Seite überstehende Ende des Zugelements (8) oder Fadens zur Gegenhalterung beispielsweise an der Herzaußenseite mit einem Gegenanker oder Knoten versehen ist.

9. Vorrichtung nach einem der Ansprüche 1 bis 8, **dadurch gekennzeichnet, dass** der auf das Zugelement (8) aufschiebbare Sensor (3) einen zusätzlichen Faden (15) trägt, mittels welchem er auch entgegen der Schieberichtung positionierbar oder wieder aus dem Herzen (2) herausziehbar ist.

10. Vorrichtung nach einem der Ansprüche 1 bis 9, **dadurch gekennzeichnet, dass** an der einen Seite des Sensors (3) das Zugelement (8) mit dem Anker (7) und an der entgegengesetzten Seite das Zugelement (15) für den Gegenanker (12) oder Knoten angreift.

11. Vorrichtung nach einem der Ansprüche 1 bis 10, **dadurch gekennzeichnet, dass** das Gehäuse (4) des Sensors (3) aus biokompatiblen Werkstoff besteht und im Verhältnis zu seiner Länge einen geringen Querschnitt hat.

12. Vorrichtung nach einem der Ansprüche 1 bis 11, **dadurch gekennzeichnet, dass** in dem Gehäuse (4) des Sensors (3) wenigstens zwei Druckaufnehmer (3) angeordnet sind, die zueinander beabstandet oder einander gegenüberliegend in oder an dem Gehäuse (4) angeordnet sind.

13. Vorrichtung nach einem oder mehreren der vorstehenden Ansprüche, **dadurch gekennzeichnet, dass** der Gegenanker (12) wenigstens einen Teil der Elektronik des Sensors (3) / der Sensoren (3) enthält.
